# EUROPEAN PATENT APPLICATION

(11) **EP 2 404 612 A1**
(43) Date of publication of application: **11.01.2012**
(21) Application number: 10168867.9
(22) Date of filing: 08.07.2010
(51) Int. Cl.: A61K 38/17, C07K 14/47

(54) **Diagnosis, prophylaxis and therapy of Alzheimer's disease and other neurodementing disorders**

(71) Applicant: Balakrishnan, Karthikeyan, 67065 Ludwigshafen (DE); Bacher, Michael, 35039 Marburg (DE)
(72) Inventor: Balakrishnan, Karthikeyan, 67065 Ludwigshafen (DE); Bacher, Michael, 35039 Marburg (DE)
(74) Representative: Dehmel, Albrecht

(57) **Abstract**

The present invention relates to a polypeptide having an amino acid sequence comprising at least two substitutions of the glycine residues in one or more GXXXG motives in the amino acid sequence of the wild type Aβ peptide as depicted in SEQ ID NO:1. Furthermore the present invention relates to methods and a kit for diagnosing a neurodementing disease. The present invention further relates to the polypeptide for use as a medicament in human and veterinary medicine and as a diagnostic substance, in particular for use as a medicament for the prevention and for the diagnosis of the progression of Alzheimer's disease, Down's syndrome, Dementia with Lewy bodies, fronto-temporal dementia, misfolded protein/peptide related diseases, cerebral amyloid angiopathy, Prion disease and amyloidoses.

## Description

The present invention relates to a polypeptide having an amino acid sequence comprising at least two substitutions of the glycine residues in one or more GXXXG motives in the amino acid sequence of the wild type human Aβ peptide as depicted in SEQ ID NO:1. Furthermore the present invention relates to methods and a kit for diagnosing a neurodementing disease. The present invention further relates to the polypeptide for use as a medicament in human and veterinary medicine and as a diagnostic substance, in particular for use as a medicament for the prevention and for the diagnosis of the progression of Alzheimer's disease, Down's syndrome, Dementia with Lewy bodies, fronto-temporal dementia, misfolded protein/peptide related diseases, cerebral amyloid angiopathy, Prion disease and amyloidoses.

Alzheimer's disease (AD), the most common form of dementia among elderly population (prevalence: 1000/100,000 >65 y), represents the fourth leading cause of death in the developed world. Cortical atrophy, neuronal loss, region-specific amyloid deposition, neuritic plaques, and neurofibrillary tangles are key neuropathological features in AD brain. These alterations are thought to be linked to cognitive decline which clinically defines AD. Within these markers, neuritic plaques are amyloid immunoreactive, thioflavin positive, and accompanied by astrogliosis, microgliosis, cytoskeletal changes, and synaptic loss. The degree of neuritic degeneration within plaques correlates with clinical parameters of dementia. Neuritic plaques are spherical, multicellular lesions that are usually found in moderate to large numbers in limbic structures and associated neocortices of the AD brain. These plaques are comprised of extracellular deposits of amyloid-β peptide(s) (Aβ) that include abundant amyloid fibrils intermixed with non-fibrillary forms of the peptide. Such plaques also contain variable numbers of activated microglia that are often situated very near the fibrillar amyloid core, as well as reactive astrocytes surrounding the core.

The major constituent of the neuritic plaque, Aβ peptides, arises from a larger precursor protein, the amyloid precursor protein (APP) (Kang, et al., 1987; Tanzi, et al., 1987). Aβ is produced by normal cells and can be detected as a circulating peptide in the plasma and cerebrospinal fluid (CSF) of healthy humans. Although the physiological role of the amyloid precursor protein (APP) in the brain is not well understood, missense mutations in APP confer autosomal dominant inheritance of AD (FAD), and shed light on potentially important pathogenic mechanism(s). The accumulation of Aβ, a 39-42 amino acid proteolytic product of APP, in neuritic plaques, which at autopsy fulfill the neuropathological criteria for a definitive diagnosis of AD, is thought to be causative for disease progression. A major Aβ cleavage product of APP is the Aβ (1-42) polypeptide, but Aβ peptides shorter at the C-terminus (39 to 41) are also produced by the proteolytic (γ-secretase) cleavage in the membrane. The N-terminal part of Aβ (1-42) is localized in the extracellular region of APP, and the major C-terminal part of the Aβ peptide is contained within the transmembrane domain.

Missense mutations, in APP associated with FAD, occur in proximity to the Aβ domain and result in an increase in the production of the 4kDa Aβ peptide. In AD, it has been postulated that increased synthesis and/or a decreased clearance of Aβ may lead to amyloid plaque deposition and subsequently to the neuropathological changes associated with the disease. *In vitro* studies, using synthetic Aβ peptide(s), have shown that neurotoxicity is dependent on Aβ being fibrillar and predominantly in a β-pleated sheet conformation.

The accumulation of extracellular plaques containing the neurotoxic amyloid peptide fragment (Aβ) of β-amyloid precursor protein (APP), as the major product, is one of the characteristics of Alzheimer's disease (AD). Although APP has been recognised as a key molecule for AD, the molecular (patho-) physiological degradation and proteolytic pathways of APP, and cellular interactions and biochemical fate of Aβ peptide(s) are still unclear.

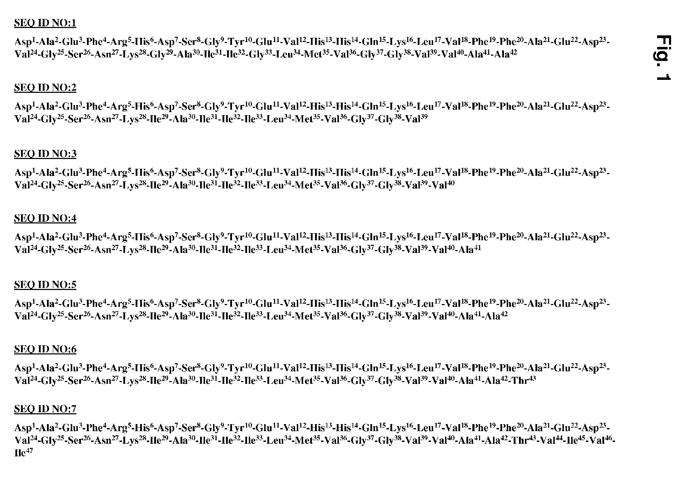

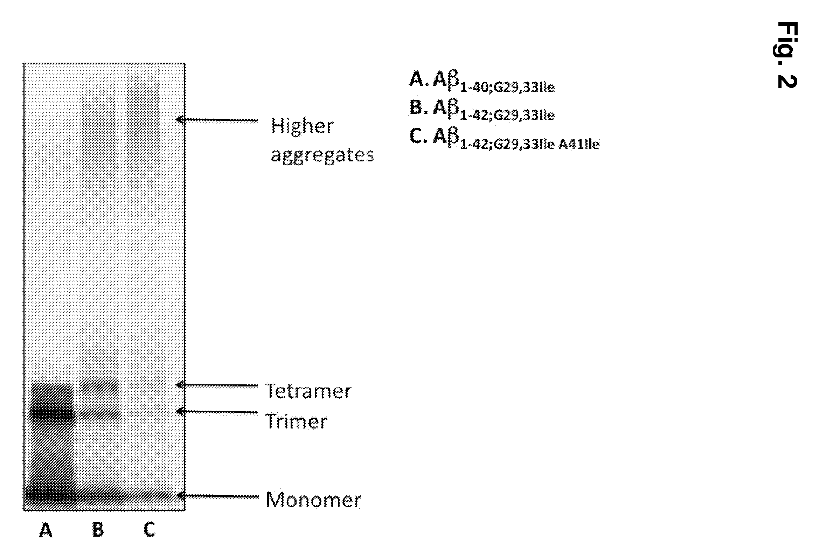

Despite the lack of details on degradation pathways and cellular transport for the formation and deposition of Aβ-derived plaques, recent studies elucidated in more detail the properties of autoantibodies which are directed against the Aβ peptide (Du et al., 2001; Dodel et al., 2002). Recently, antibodies directed against Aβ have been produced upon immunisation with Aβ(1-42) and their recognition properties were investigated in more detail. This work resulted in the identification of a specific Aβ-epitope recognised by the antibodies generated in transgenic AD mice (McLaurin et al., 2002; Przybylski et al., 2003). These results have been obtained by using selective proteolytic excision technologies (Epitope-Excision) in combination with high resolution mass spectrometry (FTICR-MS) as bioanalytical tools of high sensitivity and specificity for the identification of antigen epitopes (Macht et al 1996; Suckau et al 1992; Macht et al. 2004; see Figures 1, 2). Using mass spectrometric epitope excision of the immobilised Aβ-antigen-immune complex, the epitope was identified to consist of the residues (4-10) (FRHDSGY) of Aβ(1-42). The selectivity of this recognition structure was ascertained by elucidation of the identical epitope from AD plaques, Aβ42 extracts from Aβ-protofibrils, chemically synthesised Aβ42, and other (Aβ-independent) polypeptides comprising the N-terminal Aβ sequence (Przybylski et al. 2003).

The major current diagnostics of AD can be grouped into (i), determinations for genetic risk factors or mutations (mainly for FAD cases, but not for sporadic AD diagnostics); (ii), neuroimaging methods; and (iii), diagnostics based on biochemical/biological markers. Present work on the development of diagnostics procedures based on biomarkers have been mainly focused on, and limited to CSF, which has the principal disadvantage that such methods require elaborate, invasive material such as CSF. A major problem associated with brain-derived biomarkers is that clinically examined controls often also include subjects with preclinical AD pathology. Further, current available biomarkers have the major disadvantage of low specificity. Similar disadvantages have been noted for a series of proteins expressed preliminary in the frontal cortex, identified by brain proteomics approaches, as potential brain biomarkers arising from presumed alterations of blood brain barrier in AD.

Studies on biomarkers in plasma and serum have been performed mainly with determinations of SP and NFT components, e.g. the Aβ peptides Aβ (1-40) and Aβ (1-42) (found with elevated levels) and hyperphosphorylated Tau-protein. However the specificity of Aβ determinations, and application for early and differential diagnostics has been considered uncertain, the same is the case for protein Tau determinations which has been described as a marker of already progressing neurodegeneration.

Recent studies towards the development of immunisation methods for the prophylaxis and therapy of AD based on therapeutically active antibodies produced from Aβ(1-42) have yielded initial success in transgenic mouse models of Alzheimer's disease. Several reports have demonstrated that antibodies generated by immunisation with Aβ(1-42) are capable of inhibiting the formation of Aβ-plaques by disaggregating Aβ-fibrils, and improve the impairments in the spatial memory of mice. The transgenic APPV717F mouse (Tg mouse) is a well-characterized model of AD-like plaque pathology with age- and region-dependent deposits of Aβ(1-40) and Aβ(1-42) (Games, et al., 1995). Recently, Schenk et al. and others investigated alterations in the deposition of Aβ in APPV717F Tg mouse following immunization with pre-aggregated Aβ(1-42) or administration of antibodies against Aβ (Bard, et al., 2000; Schenk, et al., 1999). Both immunization and administration of Aβ antibodies significantly attenuated amyloid plaque deposition, neuritic dystrophy, and astrogliosis. In these studies, increased titers of mouse anti-human Aβ-antibodies were necessary for the observed reduction in plaque burden. These findings raise the possibility that formation and clearance of an Aβ-antigen: antibody complex may decrease brain Aβ deposition either following antibody generation within the central nervous system or by peripheral antibody transport across the blood-brain-barrier (BBB). Furthermore, passive immunization appears to reduce brain Aβ burden by altering Aβ equilibrium between the CNS and plasma (DeMattos, et al., 2001). Remarkably, active or passive immunization significantly reverses cognitive and memory impairment in APPV717F mouse or other APP transgenic mice (Dodart, et al., 2002; Janus, et al., 2000; Morgan, et al., 2000). These results suggest that immunization may prevent memory deficits possibly by altering a soluble pool of Aβ. Thus, treatment of AD patients with active or passive immunization is one of several emerging therapeutic approaches targeting the production, clearance, and aggregation of the Aβ peptide.

Based on these results, a clinical trial using an active immunization procedure (Aβ(1-42) peptide and/or preaggregates thereof; Adjuvans: QS21) was initiated for treatment of patients with established AD. Unfortunately, severe side-effects developed ("meningoencephalitis") and the clinical trial was stopped. A subgroup of AD patients (n=30) treated with active immunization in this clinical trial, was analyzed (Hock, et al., 2002; Hock, et al., 2003). The authors demonstrated that (i), immunization induces the production of antibodies against Aβ(1-42) and (ii), in patients where a production of antibodies was observable, the cognitive decline was significantly reduced in comparison to the untreated control group. The authors concluded that immunization may be a therapeutic option for AD.

Thus, there is a need to provide highly specific and sensitive methods for diagnosis and a vaccine with constant quality and reduced side effects for the prophylaxis of Alzheimer's disease and other neurodementing diseases.

The inventors have solved the problem by way of the subject matter set forth in the claims.

The following figures form part of the present specification and are included to further demonstrate certain aspects of the present invention. The invention may be better understood by reference to one or more of these figures in combination with the detailed description of specific embodiments presented herein.
**Figure 1****:** Amino acid sequences of the Aß peptides according to the invention (SEQ ID NOs: 1-7).
**Figure 2****:** Result of the SDS-PAGE analysis of the oligomerization state of different Aß peptides with two and three isoleucine substitutions, respectively. Gels were loaded with mutant Aβ (1-40;G29Ile,G33Ile), Aβ (1-42; G29Ile,G33Ile) and Aβ (1-42; G29Ile,G33Ile ,A42Ile) peptides. Subsequently, gels were silver stained.

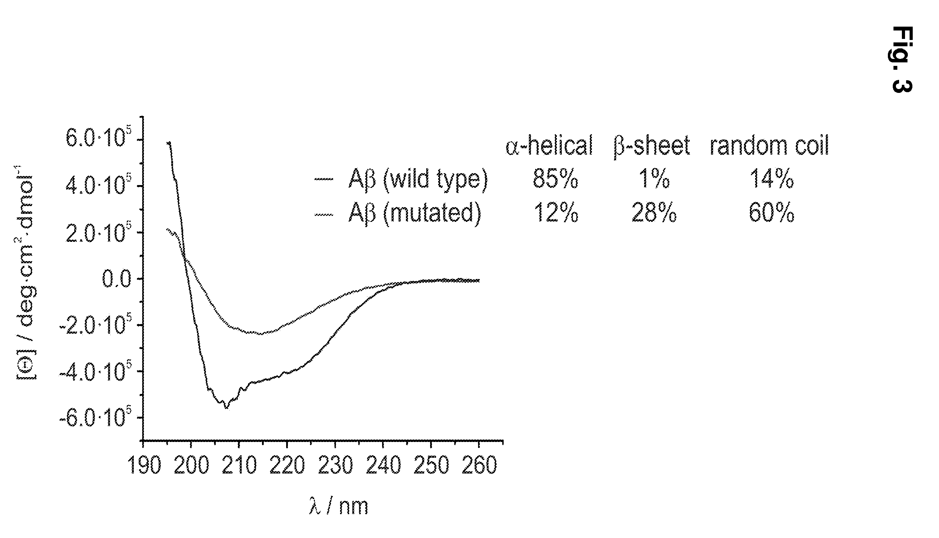

**Figure 3****:** Result of CD spectra analysis of the comparison of wild type Aβ and mutated Aβ (1-40; G29Ile,G33Ile) concerning occurrence of α-helical, ß-sheet and random coil conformation.

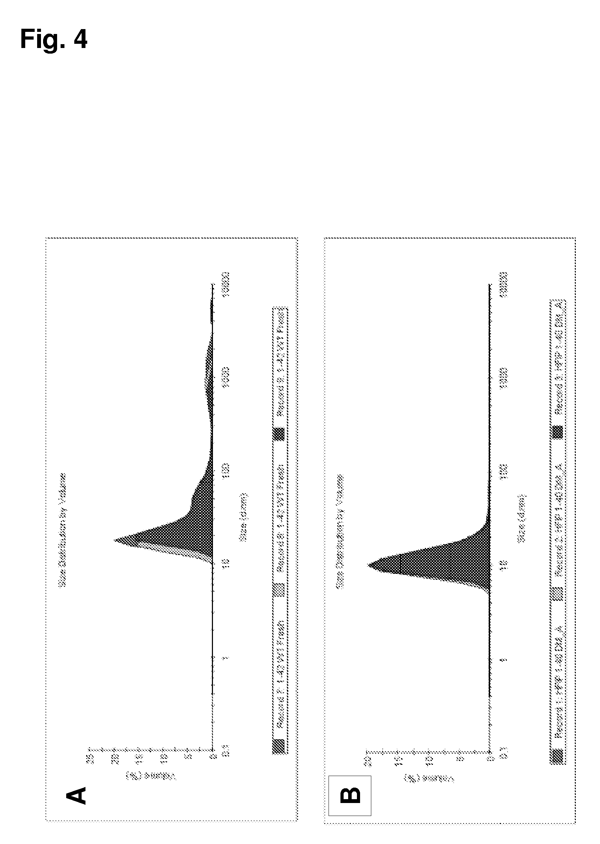

**Figure 4****:** Result of light scattering experiments of the comparison of the oligomerization state of the wild-type Aß (1-42) (Figure 4A) and the mutant Aβ (1-40;G29Ile,G33Ile) (Figure 4B) G29Ile,G33Ile. Light scattering data show the size distribution by volume denoting the number of molecules falling in a particular size range (nanometer in diameters). The three coloured peaks denote triplicate measurements of the same sample.

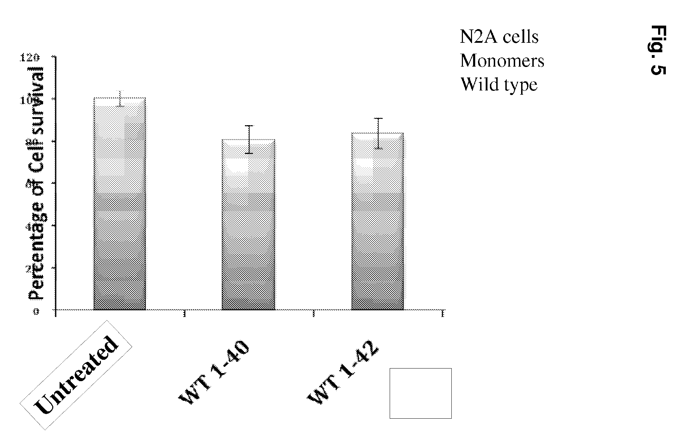

**Figure 5****:** Neurotoxicity of monomeric wild type Aβ (1-40) and Aβ (1-42) peptides on murine neuronal cell line (N2A). Cells were treated with freshly prepared monomeric Aβ (1-40) and Aβ (1-42) peptides. Bars represent percentage of cell survival after peptide treatment of the cells. The data are representative of duplicated samples and one of three experiments.

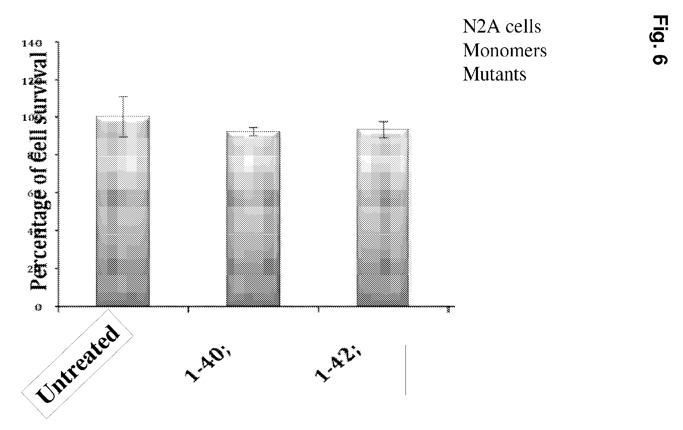

**Figure 6****:** Neurotoxicity of monomeric mutant Aβ (1-40;G29Ile,G33Ile) and Aβ (1-42;G29Ile,G33Ile) peptides on N2A cells. Cells were treated with freshly prepared Aβ (1-40;G29Ile,G33Ile) and Aβ (1-42;G29Ile,G33Ile) peptides which contained more monomers as they were pretreated with hexafluoro isopropanol (HFIP). Bars represent percentage of cell survival after peptide treatment. The data are representative of duplicated samples and one of three experiments.

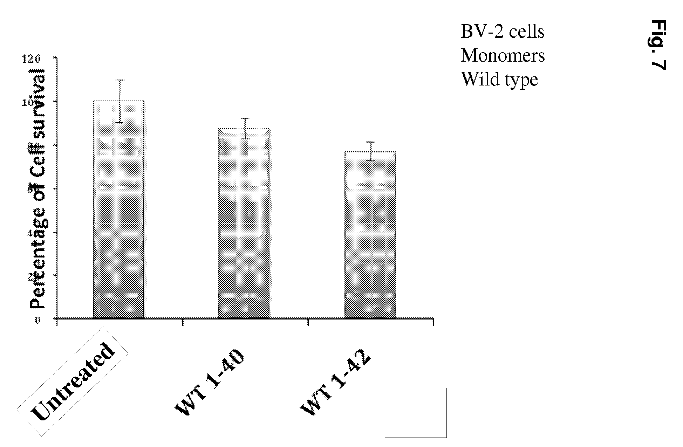

**Figure 7****:** Neurotoxicity of monomeric wild type Aβ (1-40) and Aβ (1-42) peptides on microglial cell line (BV-2). BV-2 cells were treated with freshly prepared Aβ (1-40) and Aβ (1-42) peptides which contained more monomers as they were pretreated with HFIP. Bars represent percentage of cell survival after peptide treatment. The data are representative of duplicated samples and one of three independent experiments.

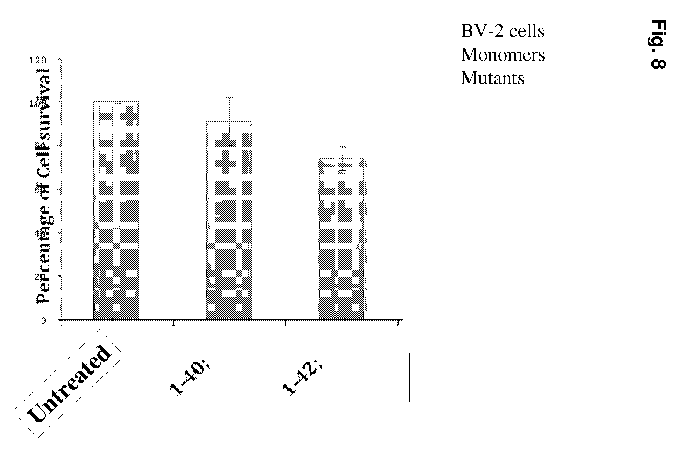

**Figure 8****:** Neurotoxicity of monomeric mutant Aβ (1-40;G29Ile,G33Ile) and Aβ (1-42;G29Ile,G33Ile) peptides on BV-2 cells. Cells were treated with freshly prepared mutant peptides Aβ (1-40;G29Ile,G33Ile) and Aβ (1-42;G29Ile,G33Ile) peptides which contained more monomers as they were pretreated with HFIP. Bars represent percentage of cell survival after peptide treatment. The data are representative of duplicated samples and one of three experiments.

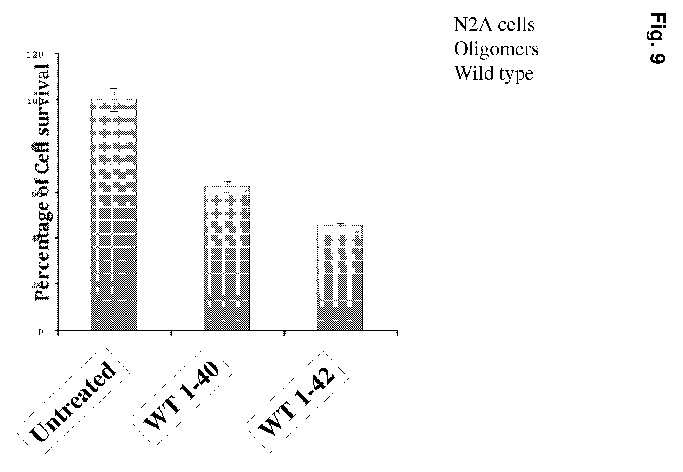

**Figure 9****:** Neurotoxicity of oligomeric wild type Aβ (1-40) and Aβ (1-42) peptides on N2A cells. Cells were treated with freshly prepared oligomers of wild type Aβ (1-40) and Aβ (1-42) peptides. Prior to treatment, the peptides were maintained under conditions which trigger oligomerization. Bars represent percentage of cell survival after peptide treatment. The data are representative of duplicated samples and one of three experiments.

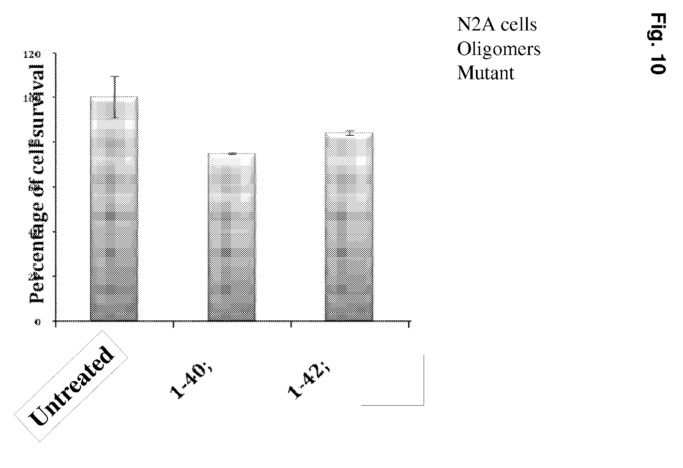

**Figure 10****:** Neurotoxicity of oligomeric mutant Aβ (1-40;G29Ile,G33Ile) and Aβ (1-42;G29Ile,G33Ile) peptides on N2A cells. Cells were treated with freshly prepared oligomers of Aβ (1-40;G29Ile,G33Ile) and Aβ (1-42;G29Ile,G33Ile) peptides. Prior to treatment on cells the peptides were maintained under conditions which trigger oligomerization. Bars represent percentage of cell survival after peptide treatment. The data are representative of duplicated samples and one of three experiments.

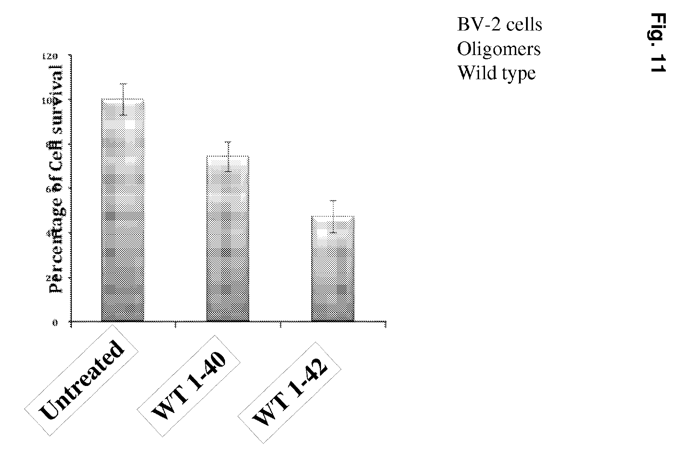

**Figure 11****:** Neurotoxicity of oligomeric wild type Aβ (1-40) and Aβ (1-42) peptides on BV-2 cells. Cells were treated with freshly prepared oligomers of wild type Aβ (1-40) and Aβ (1-42) peptides. Prior to treatment on cells the peptides were maintained under conditions which trigger oligomerization. Bars represent percentage of cell survival after peptide treatment. The data are representative of duplicated samples and one of three experiments.

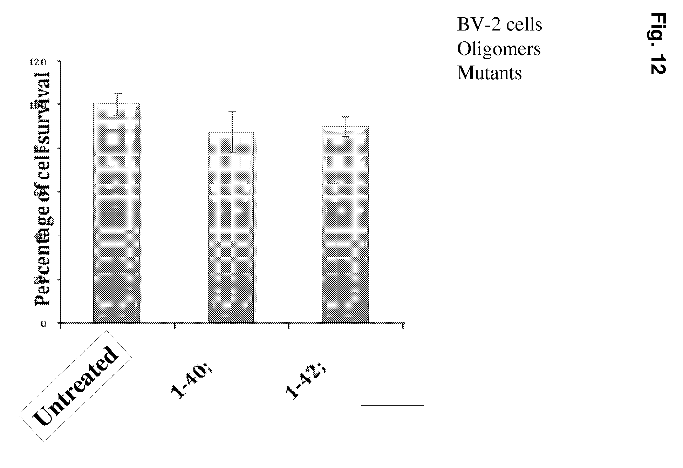

**Figure 12****:** Neurotoxicity of oligomeric mutant Aβ (1-40;G29Ile,G33Ile) and Aβ (1-42;G29Ile,G33Ile) peptides on BV-2 cells. Cells were treated with freshly prepared oligomers of Aβ (1-40;G29Ile,G33Ile) and Aβ (1-42;G29Ile,G33Ile) peptides. Prior to treatment on cells the peptides were maintained under conditions, which trigger oligomerization. Bars represent percentage of cell survival after peptide treatment. The data are representative of duplicated samples and one of three experiments.

The term "Aβ (1-Y)" refers to an Aβ peptide, which exhibits the wild type amino acid sequence according to SEQ ID NO:1 from the N-terminal position 1 to the C-terminal position Y, e.g. the term "Aβ (1-42)" refers to an Aβ peptide, which exhibits the wild type amino acid sequence according to SEQ ID NO:1 from the N-terminal position 1 to the C-terminal position 42.

The term "Aβ (G29Ile,G33Ile)" refers to an Aβ peptide having two amino acid residue substitutions at positions 29 and 33 in the wild type amino acid sequence according to SEQ ID NO:1 from glycine to isoleucine.

The term "Aβ (G29Ile,G33Ile,A42Ile)" refers to an Aβ peptide having three amino acid residue substitutions at positions 29, 33 and 42 in the wild type amino acid sequence according to SEQ ID NO: from glycine to isoleucine at positions 29 and 33 and from alanine to isoleucine at position 42.

The term "substitution", as used herein, means a replacement of a wild type amino acid residue by another amino acid residue.

The term "oligomer", as used herein, refers to a polypeptide consisting of at least two monomers. Preferably, the oligomer is a homomer, i.e. the oligomer consists of at least two identical monomers. The term "low molecular weight oligomer", as used herein, refers to dimers, trimers and/or tetramers, in particular trimers. The term "high molecular weight oligomer", as used herein, refers to pentamers and higher.

The term "autoantibody" or "autoantibodies", as used herein, refers to antibodies which are directed against epitopes on proteins of the human body and which can be found in the blood or cerebrospinal fluid of a human subject without prior immunisation with the respective antigen. The term herein particularly refers to antibodies specifically binding to the C-terminal half of Aβ polypeptide. Preferably, such binding is specific, in particular under physiological conditions, e.g. pH about 7.4, salt concentrations about 50 to about 150 mM in PBS. Preferably, the specific binding has under *in vitro* conditions a relative dissociation constant (relative K_{D}; reflecting *in vitro* results but not necessarily identical values under *in vivo* conditions) of at least about 10⁻⁵ M or lower, more preferably about 10⁻⁶ M, about 10⁻⁷ M, about 10⁻⁸, about 10⁻⁹ M, about 10⁻¹⁰ M, about 10⁻¹¹ M, about 10⁻¹² M, or even less. In a particularly preferred embodiment the relative dissociation constant of the binding of said polypeptide to said epitope is in between about 10⁻⁸ M to about 10⁻¹² M, in particular around 1 to 50 x 10⁻⁹ M.

The terms "neurodementing diseases", "dementing disorders" or "neurodementia diseases of AD-type", as used herein, refer to diseases selected from Alzheimer's disease (AD), Down's syndrome, Dementia with Lewy bodies, fronto-temporal dementia as well as to disorders such as cerebral amyloid angiopathy, misfolded protein/peptide related diseases, Prion disease and amyloidoses.

The term "moiety", as used herein, refers to a portion of a polypeptide with distinct function(s). Such moieties can provide for a structural or functional feature which is normally not present in the rest of the polypeptide or which feature is enhanced by this moiety. Such functional or structural moieties can for example provide binding, stabilization or detection of the polypeptide. The moiety can be a polypeptide on its own or can be any other compound, which provides the desired function(s) to the polypeptide. Said moiety is stably associated with the polypeptide, in particular covalently coupled to the polypeptide. The term moiety, as used herein, does not confer any information about the size of this portion in comparison to the polypeptide itself. The moiety can be smaller, equally sized or larger than the polypeptide it is coupled to.

The present invention relates to a polypeptide having an amino acid sequence comprising at least two substitutions of the glycine residues in one or more GXXXG motives in the amino acid sequence of the wild type human Aβ peptide. The wild type Aβ peptide exhibits preferably the amino acid sequence as depicted in SEQ ID NO:1. However, the polypeptide according to the present invention may exhibit more or less amino acid residues than the amino acid sequence as depicted in SEQ ID NO:1. E.g. the polypeptide according to the present invention consist of the first 29, 30, 31, 32, 33, 34, 35, 36, 37 or 38 amino acid residues of according to SEQ ID NO:1 and having at least two substitutions of the glycine residues in one or more GXXXG motives. Furthermore, the polypeptide according to the present invention may consist of 43 or 47 amino acid residues.

In the wild type amino acid sequence according to SEQ ID NO:1, the GXXXG motives are located at the positions 25-29, 29-33 and 33-37. Thus, the at least two substitutions may occur at any of said GXXXG motives, namely G25XXXG29, G29XXXG33 and/or G33XXXG37. Further, the at least two substitutions can occur within different GXXXG motives, e.g. at the glycine residues at the positions 25 and 33, 25 and 37 or 29 and 37 according to the wild type amino acid sequence of the Aβ peptide.

In a more specific embodiment the substitutions occur at the glycine residues at positions 29 and 33 of the wild type amino acid sequence of the Aβ peptide as depicted in SEQ ID NO:1.

Preferably, the glycine residues of the GXXXG motive are replaced by hydrophobic amino acid residues, in particular by isoleucine.

Preferably, the polypeptide according to the present invention exhibits an amino acid sequence as depicted in any one of SEQ ID NOs: 2-7.

The polypeptide according to the present invention is able to instantly form stable low-molecular weight oligomers, in particular trimers. Forming of dimers is substantially prevented by the at least two substitutions introduced into the polypeptide according to the present invention. Low-molecular weight oligomers, in particular trimers are thought to play a critical role in the onset of AD.

The at least two substitutions introduced in the polypeptide according to the present invention are further able to increase its β**-**sheet conformation. Taken together, the at least two substitutions lead to the instant formation of ß-sheet enriched stable low-molecular weight oligomers, in particular trimers. It has been shown that polypeptides, in particular naturally occurring autoantibodies, which specifically bind to the wild type Aß peptide, bind more specific and more sensitive to the polypeptide according to the present invention. Consequently, the more specific and more sensitive binding of polypeptides, in particular to the polypeptide according to the present invention can effectively be used in the diagnosis of Alzheimer's disease and other neurodementing diseases.

In another specific embodiment, the polypeptide according to the present invention can further comprise additional amino acid residues. For example, tags, markers, binding domains, activation domains or similar functional moieties can be fused to the Aβ sequence stretch, for instance, to provide for a better binding of the polypeptide according to the present invention to certain surfaces. Analogous, the polypeptide according to the present invention can be modified for certain purposes, such as covalent coupling to a fluorophor or chromophor, biotin, or the like.

In a preferred embodiment the above mentioned polypeptide additionally comprises one or more moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids.

In another aspect the present invention relates to a method for diagnosing a neurodementing disease, the method comprising the following steps:
a) incubating a polypeptide according to the present invention immobilized on a carrier with a sample derived from a subject, subsequently
b) separating said sample from the carrier, and
c) detecting polypeptides bound to the immobilized polypeptide of step a).

The present invention also relates to a method for diagnosing a neurodementing disease, the method comprising the following steps:
a) incubating a polypeptide according to the present invention with a sample derived from a subject, and subsequently
b) incubating a carrier with the sample, the carrier having a binding affinity for the polypeptide according to the present invention,
c) separating said sample from the carrier, and
d) detecting polypeptides bound to the polypeptide according to the present invention, said polypeptide being bound to the carrier.

In a preferred embodiment detection of polypeptides in step c) and d), respectively, is performed by means of ELISA, ELISPOT, Western-Blot, Dot Blot, Protein-Chip, surface plasmon resonance assay, immunoprecipitation or co-immunoprecipitation, or affinity chromatography, in particular immunoaffinity chromatography.

The present invention also relates to a method for diagnosing a neurodementing disease, the method comprising the following steps:
a) incubating a polypeptide according to the present invention immobilized on a carrier with a cell containing sample derived from a subject,
b) separating said sample from the carrier, and
c) detecting cells bound to the immobilized polypeptide of step a).

The present invention also relates to a method for diagnosing a neurodementing disease, the method comprising the following steps:
a) incubating a polypeptide according to the present invention with a cell containing sample derived from a subject, and subsequently
b) incubating the sample with a carrier having a binding affinity for a polypeptide of the present invention,
c) separating said sample from the carrier, and
d) detecting cells bound to the polypeptide according to the present invention, said polypeptide being bound to the carrier.

In a preferred embodiment the methods of the invention comprise an additional step, wherein at least one washing step is performed before the detecting step.

In a preferred embodiment of the methods according to the present invention, relating to the detection of cells, the methods are carried out in form of an affinity chromatography, in particular immunoaffinity chromatography.

The amount of polypeptides, e.g. antibodies, binding to a polypeptide according to the present invention in a sample of a subject is an indicator for the status of the subject with regard to the development and/or progression of AD. The higher the concentration of polypeptides directed against the polypeptide according to the present invention, the higher the protective capacity and the lower the risk of development and/or progression of AD. For diagnostic reasons, the methods of detection according to the present invention can thus be performed individually, but also in combination in order to provide for a more specific diagnosis.

In a preferred embodiment the methods according to the invention are carried out for diagnosing Alzheimer's disease, Down's syndrome, Dementia with Lewy bodies, fronto-temporal dementia, cerebral amyloid angiopathy, and/or amyloidoses.

In a further aspect, the present invention relates to a carrier comprising a polypeptide according to the present invention. The carrier according to the invention and used in the methods of the invention can be of any suitable material, capable of binding polypeptides, such as beads, in particular magnetic beads or sepharose beads, membranes, in particular polyvinylidene fluoride or nitrocellulose membranes, glass, sepharose matrices, gold surfaces, synthetic surfaces, in particular microtiter plates. For certain embodiments, the surface of the carriers can be coated with agents, which are, for instance, capable of binding to the tags and markers mentioned above.

In another aspect, the present invention relates to a kit for the diagnosis of a neurodementing disease, wherein the kit comprises a polypeptide according to the present invention.

In a preferred embodiment the sample or the cell containing sample, respectively, used for the methods of the present invention is derived from blood, plasma, urine or cerebrospinal fluid (CSF) of a subject. In an even more preferred embodiment the cell containing sample is derived from blood and the cells are of the B-cell lineage. The sample, i.e. the subject can be of human, rodent, bovine, porcine, canine or avian origin. In particular the sample or the cell containing sample can be derived from human, mouse, rat, rabbit, cow, pig, dog, chicken and so forth.

In a preferred embodiment the sample or the cell containing sample, respectively, used for the methods of the present invention is derived from a tissue of a subject. In an even more preferred embodiment the sample or the cell containing sample is derived from brain, e.g. brain slices. The sample, i.e. the subject can be of human, rodent, bovine, porcine, canine or avian origin. In particular the sample or the cell containing sample can be derived from human, mouse, rat, rabbit, cow, pig, dog, chicken and so forth.

In a preferred embodiment the polypeptide according to the present invention additionally comprises other moieties such as tags or markers, which facilitate in particular the attachment of the polypeptide according to the invention to a carrier. In particular such tags can provide for the immobilisation of the polypeptide on a carrier coated with the respective antagonist. Examples for such moieties are biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids.

The above mentioned tags or markers can be directly fused to the polypeptide according to the present invention. If a higher flexibility between the tags or markers and the polypeptide according to the present invention is desired, linkers can be introduced. Such linkers can be for instance polyglycine or - alanine linkers. Biotin, a non-peptidic substance, can be covalently linked to a polypeptide of the present invention. A multitude of possible combinations of markers and carriers for the immobilisation of a polypeptide of the present invention is known from the prior art.

In a preferred embodiment the polypeptide according to the present invention is directly synthesized by conventional polypeptide synthesis methods.

Alternatively, the polypeptide according to the present invention can also be obtained by *in vitro* translation or via a recombinant expression system. In order to express a polypeptide according to the present invention a respective nucleic acid expression construct has to be generated. A person skilled in the art will clearly see several ways to construct appropriate expression system harbouring a nucleic acid sequence encoding for a polypeptide according to the present invention. The construct is subsequently expressed in a suitable host cell and the polypeptide is isolated. For this purification step the above mentioned markers and/or tags can be used as well.

In a preferred embodiment, the methods according to the invention comprising the detection of polypeptides in step c) or d), respectively, represent an ELISA. In this case the carrier is for example a microtiter plate. The separation of sample and carrier is achieved by removing the sample liquid from the microtiter plate and/or by washing the microtiter plate after the incubation. Preferably, the bound polypeptide is in this scenario an antibody and this antibody can be detected for example via a secondary antibody, coupled with e.g. alkaline phosphatase (AP), and the addition of a substrate for AP resulting in the turn over of the substrate into, for example, a coloured compound detectable by an optical device. A person skilled in the art and familiar with ELISA techniques will know several variations of the ELISA concept, which can be applied to the methods of the present invention as well.

Alternatively, in another preferred embodiment, the methods according to the invention comprising the detection of polypeptides in step c) or d), respectively, represent an ELISPOT assay. In this case the carrier is for example a nitrocellulose plate. The incubation step of the carrier with the sample provides in this scenario enough time for a cell in the sample to produce sufficient amounts of antibody. The separation of sample and carrier is achieved by removing the sample liquid from the nitrocellulose plate and/or by washing the nitrocellulose plate after the incubation. Preferably, the bound polypeptide is an antibody and this antibody can be detected, for example via a secondary antibody, coupled with e.g. alkaline phosphatase (AP), and the addition of a substrate for AP, such as BCIP/NBT (Bromo-chloro-indoryl phosphate / Nitro Blue Tetrazolium) resulting in the turn over of the substrate into, for example, a deep purple stain detectable visually or by an optical device. A person skilled in the art and familiar with ELISPOT techniques will know several variations of the ELISPOT concept, which can be applied to the methods of the present invention as well.

In a further embodiment, the methods according to the invention comprising the detection of polypeptides in step c) or d), respectively, represent a Western Blot or Dot Blot assay. In this case the carrier is for example a nitrocellulose membrane. For the Western Blot, on the nitrocellulose membrane is either immobilized a polypeptide as used in step a) of the methods according to the present invention or a substance with binding affinity for a polypeptide as used in step a) of the methods according to the present invention. The nitrocellulose or similar membrane (PVDF etc.) itself can provide for the binding affinity to the polypeptide as used in step a) of the methods according to the present invention. The separation of sample and carrier is achieved by removing the sample liquid from the nitrocellulose membrane and/or by washing the nitrocellulose membrane after the incubation. Preferably, the bound polypeptide is an antibody and this antibody is for example detected via a labelled secondary antibody, e.g. coupled with horseradish peroxidase, and subsequent luminescent reaction and detection. A person skilled in the art and familiar with Western Blot/Dot blot techniques will know several variations of the Western Blot/Dot blot concept, which can be applied to the methods of the present invention as well.

In a further embodiment, the methods according to the invention comprising the detection of polypeptides in step c) or d), respectively, represent a Protein chip, i.e. protein microarray. In this case the carrier is for example a glass surface functionalized for binding proteins. The separation of sample and carrier is achieved by removing the sample liquid from the glass carrier and/or by washing the glass carrier after the incubation. Preferably, the bound polypeptide is an antibody and this antibody is detected via a labelled secondary antibody, coupled with e.g. a fluorescent dye, which can be detected by an optical device. A person skilled in the art and familiar with protein chip techniques will know several variations of the protein chip concept, which can be applied to the methods of the present invention as well.

In a further embodiment, the methods according to the invention comprising the detection of polypeptides in step c) or d), respectively, represent a surface plasmon resonance analysis. In this case the carrier is for example a metal surface such as a gold surface. The separation of sample and carrier is achieved by removing the sample liquid from the metal carrier and/or by washing the metal carrier after the incubation. Preferably, the bound polypeptide is an antibody and the binding of the antibody is detected via measuring the intensity of the reflected light at a specific incident angle with an optical device. A person skilled in the art and familiar with plasmon resonance analysis techniques will know several variations of the surface plasmon resonance concept, which can be applied to the methods of the present invention as well.

In a further embodiment, the methods according to the invention comprising the detection of polypeptides in step c) or d), respectively, represent a Isothermal calorimetry analysis. A person skilled in the art and familiar with Isothermal calorimetry techniques will know several variations of the Isothermal calorimetry concept, which can be applied to the methods of the present invention as well.

In a further embodiment, the methods according to the invention comprising the detection of polypeptides in step c) or d), respectively, represent a pull down or immunoprecipitation experiment. In this case the carrier may consist of sepharose beads. This sepharose beads are coated for example with gluthation (for pull down assays) or with an antibody (immunoprecipitation assays). Separation of sample and carrier is achieved by removing the sample liquid from the sepharose beads and/or by washing the sepharose beads after the incubation. Preferably, the bound polypeptide is an antibody and this antibody is detected via a subsequent Western Blot analysis of the precipitated protein complexes. A person skilled in the art and familiar with Pull down/Immunoprecipitation techniques will know several variations of these concepts, which can be applied to the methods of the present invention as well. One such variation would be the application of a co-immunopreciptation approach, wherein the carrier has only an indirect binding affinity for the polypeptide as used in step a) of the methods according to the present invention.

In a further embodiment, the methods according to the invention comprising the detection of polypeptides in step c) or d), respectively, are carried out in form of an affinity chromatography. In this case the carrier is the matrix, e.g. sepharose within a conventional chromatography column, to which is either linked a polypeptide as used in step a) of the methods according to the present invention or a substance with binding affinity for a polypeptide as used in step a) of the methods according to the present invention. The incubation of the sample with the carrier comprises the time frame the sample needs to pass through the column. The separation of sample and carrier is achieved by eluting the sample liquid from the column and/or by washing the column after the incubation. Preferably, the bound polypeptide is an antibody and this antibody is for example detected by elution of the bound antibody, for instance with a buffer having a high salt content, and subsequent detection of eluted polypeptide, for example by direct optical determination or by subsequent Western blot or similar analyses. A person skilled in the art and familiar with affinity chromatography techniques will know several variations of the affinity chromatography concept, which can be applied to the methods of the present invention as well. One such variation would be the application of an immunoaffinity chromatography approach, wherein the carrier is coated with antibodies directed against a polypeptide as used in step a) of the methods according to the present invention.

In one embodiment, the methods according to the invention comprising the detection of cells in step c) or d), respectively, are carried out in form of an affinity chromatography. In this case, for example, magnetic beads coated with sepharose represent the carrier, to which either a polypeptide as used in step a) of the methods according to the present invention is linked or a substance with binding affinity for a polypeptide as used in step a) of the methods according to the present invention. The separation of sample and carrier is achieved by eluting the sample liquid from the column and/or by washing the column after the incubation. The cells bound to the matrix are for example B- or T-cells, which can be detected, after elution of the cells from the matrix, for example by way of flow cytometry. A person skilled in the art and familiar with affinity chromatography and flow cytometry techniques will know several variations of these concepts, which can be applied to the methods of the present invention as well.

Thus, the methods according to the present invention can be carried out in particularly preferred embodiments as ELISA, ELISPOT, Western-Blot, Protein-Chip, surface plasmon resonance assay, isothermal calorimetry, immunoprecipitation or co-immunoprecipitation, or affinity chromatography, in particular immunoaffinity chromatography. These are all exemplifications of diagnostic assays. In diagnostic procedures based on surface plasmon resonance (SPR) the detection, and quantification and analysis of binding kinetics of Aβ-epitope specific antibodies can be performed by binding of (i) a biotinylated polypetide according to the invention to a avidin/streptavidin-coated SPR chip surface, or by binding (ii) polypetides according to the invention with an N-terminal Thiol-group containing carboxylic acid spacer to a gold-chip surface; followed by binding and determination of the Aβ-autoantibodies. A person skilled in the art will readily know how to incorporate the methods according to the present invention into one of these standard techniques and procedures mentioned above.

It has to be understood, that although the methods according to the present invention can be carried out in form of one of the above mentioned detection techniques per se (ELISA, ELISPOT, Western-Blot, Dot Blot, Protein-Chip, surface plasmon resonance assay, isothermal calorimetry, immunoprecipitation, affinity chromatography, etc.), it is also possible to combine these detection techniques or to apply them only for the detection step according to the invention, i.e. step c) or d), respectively, while the other steps are carried out in other formats. It is also obvious to a person skilled in the art, that the information/signals obtained in the detection steps in the methods of the present invention can provide the basis for quantification of this information/signals.

As used in this invention, an immobilized polypeptide refers in this regard to a polypeptide, which is coupled to a carrier. The coupling can be covalently or non-covalently, it can be directly to the carrier or via a linker/linking substance. If the immobilization occurs non-covalently, then the carrier or the linking substance exhibits a specific binding affinity for the polypeptide according to the present invention and vice versa. Binding affinity refers to a property of a substance, in particular a polypeptide, to associate with (an) other substance(s) and to form a stable specific dimeric or multimeric complex. Such associations rely usually on Van der Waals- or hydrogen-bonds.

The incubating step(s) serves the purpose two partners of a binding pair, i.e. having a binding affinity for each other, can associate and form a stable complex. The temperature of the incubation step may vary, but is usually from about 0°C to about 40°C, preferably from about 4 to about 37°C, even more preferred about 4°C, about 16°C, about 21°C or about 37°C. The higher the temperature, the shorter the time of incubation might be. For example, if the incubation temperature is 4°C it should last for at least 12h or over night, while 1h is usually enough for incubation at 37°C. If suitable, the carrier can be blocked prior to the method with a suitable blocking agent, reducing the likelihood of unspecific binding events. Blocking agents can be for example milk powder, BSA, fetal calf sera, or any other blocking reagent.

The detection of polypeptides bound to an immobilized polypeptide of the invention can be performed by several means. One possibility would be for example the identification via mass spectrometrical means, for example MALDI-TOF, SELDI, ESI-MS, MS-FTICR. To this purpose, immunoglobulins are first isolated from, for example, a serum sample of an AD patient or a healthy subject by protein G affinity chromatography, and subsequently Aβ-autoantibodies and Aβ-plaque specific antibodies are e.g. isolated by Aβ-epitope-chromatography, respectively. The antibodies are then immobilised, for example, on a sepharose carrier as described in the examples. The specific Aβ-epitopes, are then identified after binding of full-length-Aβ-polypeptide, followed by proteolytic epitope-excision mass spectrometric analysis using one or several of the proteases, trypsin, chymotrypsin, Glu-C protease, Asp-N-protease. After washing the affinity-bound Aβ-epitope(s) until no signal is detected in the supernatant, the specific Aβ-epitope is eluted from the column by treatment with, typically, 0.1% trifluoroacetic acid, and identified by accurate determination of its protonated molecular ions; the latter molecular ion mass accuracy is entirely sufficient for identification, but can be further ascertained by collision-induced fragmentation and tandem-MS analysis of fragment ions.

For certain embodiments, secondary antibodies labelled with a fluorescent dye or moiety (e.g. GFP) or labelled with an enzymatically active substance such as horseradish peroxidase, alkaline phosphatase, β-galactosidase or other related enzymes capable to convert a colourless substrate to a suitable dye or fluorescent product can be applied. The detection can also be accomplished by detecting the amount of occupied binding sites, i.e. utilizing a labelled autoantibody, which is incubated with the carrier after the removal of the sample.

A sample derived from a subject is derived from tissue or body fluid of a subject. The subject can be a healthy individual, i.e. not suffering from AD, or a "patient" suffering from a neurodementing disorder. In a preferred embodiment the sample or the cell containing sample, respectively, used for the methods of the present invention is obtained from blood, plasma, spleen, urine or cerebrospinal fluid (CSF) of a subject. In an even more preferred embodiment the cell containing sample is obtained from blood and the cells are of the B-cell lineage. The sample, i.e. the subject can be of human, rodent, bovine, porcine, canine or avian origin. In particular the sample or the cell containing sample can be derived from human, mouse, rat, rabbit, cow, pig, dog, chicken and so forth. Possible preparation procedures of such samples are well known from the prior art.

The β-sheet conformation of the wild type Aβ peptide is thought to be responsible for the high neurotoxicity and therefore plays a role in promoting the onset of AD. In contrast, the β-sheet conformation of the polypeptide according to the present invention exhibits a significantly decreased neurotoxicity compared to its wild type counterpart. The low-toxic polypeptide according to the present invention mimics the harmful wild type Aß peptide and is therefore suitable for therapeutic applications, e.g. active immunization or the production of antibodies for passive immunization or the production of a specific antibody for the development of an appropriate therapy against AD. Due to its low-toxic properties, the polypeptide according to the present invention significantly reduces side effects as compared to wild type Aß when applied to a patient, e.g. for the purpose of active immunization. Additionally, due to its high β-sheet conformation the polypeptide according to the present invention improves oligomer specific antibody titer when applied to patient compared to the wild type Aβ peptide, e.g for the purpose of active immunization.

In one aspect, the polypeptide according to the present invention is used as a medicament in human and veterinary medicine.

In particular, the polypeptide according to the present invention is used as a medicament in the treatment and/or prevention of the progression of Alzheimer's disease, Down's syndrome, Dementia with Lewy bodies, fronto-temporal dementia, cerebral amyloid angiopathy, Prion disease and/or amyloidoses.

Furthermore, the present invention relates to the use of a polypeptide comprising an amino acid sequence of an Aβ peptide as set forth above for use in human and veterinary medicine, in particular for use in the treatment and/or prevention of Alzheimer's disease, Down's syndrome, Dementia with Lewy bodies, fronto-temporal dementia, Prion diseases, diseases due to misfolded peptides/proteins, cerebral amyloid angiopathy, and amyloidoses.

It has been shown that in patients with Alzheimer's disease naturally occurring autoantibodies are declined compared to healthy individuals. Therefore an active immunization with the polypeptide according to the present invention can lead to an increased level of naturally occurring autoantibodies, thereby treating and/or preventing Alzheimer's disease. Additionally, a passive immunization is also possible with the antibody or a fragment thereof binding specifically to the polypeptide according to the present invention thereby treating and/or preventing Alzheimer's disease.

Administration of medicaments according to the present invention can be achieved via any common route. Although, intradermale, subcutaneous or intramuscular administration is a preferred embodiment, other routes of administration are contemplated. This includes intraperitoneal, oral, nasal, buccal, rectal, vaginal or topical.

In another preferred embodiment the medicament according to the present invention comprising a polypeptide according to the invention is administered in combination with an adjuvant, which enhances the immunogenicity of the medicament. As an adjuvants can be used , e.g. an aluminum compound, water and vegetable or mineral oil emulsions, liposomes, water-soluble glasses, microbubbles used as contrast agents in echocardiography, polyanions, non-toxic lipopolysaccharide analogues, muramyl dipeptide, and immunomodulating substances, e.g., interleukins 1 and 2, or combinations thereof.

The following examples explain the invention but are not considered to be limiting. Unless indicated differently, molecular biological standard methods were used, as e.g., described by Sambrock and Russel, 2001, Molecular cloning: A Laboratory Manual, 3. edition, Cold Spring Harbor Laboratory Press, Cold Spring Harbor, New York.

Three mutant peptides Aβ (1-40;G29,3311e), Aβ (1-42;G29Ile,G33Ile) and Aβ (1-42;G29Ile,G33Ile,A42Ile) were used to analyse their properties compared to the corresponding wild type peptides. The mutant peptides were purchased from PSL GmbH (Heidelberg, Deutschland). Oligomerization was triggered as described by Kayed et al. (Science 2003 Apr 18; 300(5618):486-9).

### Example 1: Comparison of oligomerization state of mutated Aβ peptides:

One of the classical properties of Aβ is forming the SDS-stable oligomers. SDS-PAGE could be considered as one of the confirmation of the oligomerization state.
For this purpose SDS-PAGE was performed and loaded with mutant peptides Aβ (1-40;G29Ile,G33Ile), Aβ (1-42;G29Ile,G33Ile) and Aβ (1-42;G29Ile,G33Ile,A42Ile) to demonstrate the different pattern of oligomerization state of the peptides. The samples (250 ng) were dissolved in sample buffer (Invitrogen, Carlsbad, USA) freshly before electrophoresis without any incubation steps. SDS-PAGE separation of the peptides was performed on a 10-20 % Tricine gel (Invitrogen), using a BIO-RAD Protean-(II) Electrophoresis cell, by application of approximately 250 ng peptide per lane. Subsequently, the gels were silver-stained as per manufacturer's instructions (Silver Xpress, silver staining kit, Pierce, Rockford, USA).
The double replacement within Aβ (1-40;G29Ile,G33Ile) results in less formation of high aggregates compared to the Aβ (1-42; G29Ile,G33Ile) with the double substitution and Aβ (1-42; G29Ile,G33Ile,A42Ile) with triple substitution. These data suggest that the double replacement with isoleucine at positions 29 and 33 forms instant SDS-stable enriched trimers along with monomers and tetramers and slows down further higher oligomerization.
In contrast to the mutant peptides, the Aß 1-40 wild type does not form trimers or higher oligomers under the same conditions. Even after incubation, it forms dimeric structures, which Aβ (1-40;G29Ile,G33Ile) skips completely. Instead Aβ (1-40;G29Ile,G33Ile) forms low molecular weight oligomers, in particular trimers.

### Example 2: Comparison of ß-sheet conformation of wild type and mutant Aβ peptides.

ß-sheet conformation of wild type and mutant Aβ peptides were analysed by CD spectra analysis, since enriched ß-sheet conformation is one of the key features for Aβ pathogenesis. To compare the ß-sheets formed by the wild type and the mutated trimer, respectively, CD spectra analysis was performed. Both peptides were measured at a concentration of 62.5 µM in sodium carbonate (0.05 mM)/ SDS (37.5 mM) buffer with a JASCO J-810 spectroplarimeter (JASCO, Easton, MD, USA). Far-UV CD spectra (190-260 nm) were recorded at 20°C using a quartz cuvette with an optical path length of 1 mm. The scanning speed was set at 20 nm/min with a response time 4 second and bandwidth 2 nm.
The results indicate that the mutant instantly forms ß-sheet (28%) compared to wild type Aβ (1%) after resuspending in buffer.

### Example 3: Analysis of formation of low molecular weight oligomers of Aβ (1-40;G29Ile,G33Ile) peptides.

To demonstrate that Aβ (1-40;G29Ile,G33Ile) does not form high molecular weight oligomers compared to the wild type peptide, dynamic light scattering experiments were performed. Particle size was determined using Zetasizer Nano DLS (Malvern Instruments, German). Briefly, peptides were resuspended with the respective buffers and filtered using 0.22 µM membrane filters. Measurements were performed immediately using Zetasizer Nano equipped with a 4 mW He-Ne laser at a wavelength of 633 nm at 25°C. Scattered light was detected at a 173° backward scattering angle. For data analysis, the viscosity and refractive index of water at 25°C was used. Hydrodynamic diameters of the peptides were determined in disposable cuvettes (UVette, Eppendorf, Germany).
The size distribution by volume data suggest that Aβ (1-40; G29Ile,G33Ile) peptides gives a single peak which denotes the purity of the trimeric species (99% during the measurement)..

### Example 4: Analysis of formation of low molecular weight oligomers of wild typeAβ (1-42) peptides.

To compare the data of the mutant peptides (Example 3) with the wild type peptides, light scattering studies with 8 hours incubation of the peptides were performed. The wild type Aß (1-40) hardly forms any oligomers under these conditions. As the wild type Aβ (1-42) is in contrast able to form oligomers, the mutant Aβ (1-40;G29Ile,G33Ile) and the wild type Aβ (1-42) were compared in this experiment.
Lyophilized Aβ 1-42 peptides were first resuspended in MQ water followed by addition of PBS to bring the concentration to 1 mg/ml. The prepared samples were incubated at 37°C for 8 hours with constantly shaking (900 rpm). Samples were filtered using 0.2 µM filter to avoid the fibrils and other high molecular weight contaminants.
The size distribution by volume data show a broad range of peaks of the wild type Aβ (1-42) peptide, suggesting more than one species of Aβ (1-42), whereas the results of Examples 3 lead to the conclusion that the double mutant Aβ (1-40;G29Ile,G33Ile) instantly forms in particular trimerc species.

### Example 5: Analysis of the neurotoxicity of different wild type Aβ peptides compared to different mutant Aβ peptides.

Neurotoxicity of wild type and mutant Aβ (1-40) and Aβ (1-42) as monomers and oligomers, respectively, were analyzed to estimate the harmfulness of the peptides according to the present invention on two different cell lines. As shown in example 3, the oligomeric species as used in these neurotoxicity experiments is the trimeric species (99%).
Aβ induced cytotoxicity was assessed by monitoring the conversion of the dye 3-(4,5-dimethylthiazol-2-yl)-2,5-diphenyltetrazolium bromide (MTT) to formazan. The reduction of MTT is catalyzed by mitochondrial dehydrogenase enzymes and is therefore an index for cell viability. Briefly, cells (1 ml/well) were seeded at seeding density 1x10⁵ for N2A (murine neuronal cell line) cells/ml and 1x10³ BV-2 (microglial cell line) cells/ml into 48 well plates and allowed to adhere for 24 hours. After one day the medium was replaced with F12 medium without FCS (with NEAA for N2A cells) and incubated for 48 hours. Aβ peptides were dissolved in F12 medium freshly prior to feeding the cells. Cell viability was assessed with MTT (5 mg/ml) diluted with warm cell culture medium. Cells were treated with the MTT and incubated for 4 h. Medium was carefully aspirated and washed with PBS. DMSO was added after the washing and incubated for 30 min in dark at room temperature. The absorbance measured at 570 nm with reference wavelength 750 nm with DMSO. Only the inner rows of the 48 well plates were used for the studies to minimise cell growth variations due to different medium evaporation rates at the periphery.
a) N2A cells were treated with freshly prepared wild type Aβ (1-40) and Aβ (1-42), which contained more monomers as the peptides were pretreated with hexafluoro isopropanol (HFIP).
   Treatment of N2A cells with freshly suspended monomeric wild type Aβ (1-40) and Aβ (1-42) peptides showed a maximum of 20% toxicity with monomeric wild type Aβ (1-40) at 5 µM concentration and a maximum of 18% toxicity with monomeric wild type Aβ (1-42) at 5 µM concentration when compared to the untreated cells.
b) N2A cells was treated with freshly prepared mutant Aβ (1-40;G29,33I) and Aβ (1-42;G29,33I), which contained more monomers as the peptides were pretreated with hexafluoro isopropanol (HFIP).
   Treatment of N2A cells with freshly suspended monomeric mutant Aβ (1-40;G29,33I) and Aβ (1-42;G29,33I) peptides showed a maximum of 8% toxicity with monomeric mutant Aβ (1-40;G29,33I) at 5 µM concentration and a maximum of 6% toxicity with monomeric mutant Aβ (1-42;G29,33I) at 5 µM concentration when compared to the untreated cells. The toxicities exerted by monomeric mutant peptides are comparitively weaker than that of the monomeric wild type peptides (item a) above). This implies that the two C-terminal substitutions at positions 29 and 33 minimize the toxic effect of the peptides.
c) BV-2 cells were treated with freshly prepared wild type Aβ (1-40) and Aβ (1-42), which contained more monomers as the peptides were pretreated with hexafluoro isopropanol (HFIP).
   Treatment of BV-2 cells with freshly suspended monomeric wild type Aβ (1-40) and Aβ (1-42) peptides showed a maximum of 12% toxicity with monomeric wild type Aβ (1-40) at 5 µM concentration and a maximum of 27% toxicity with monomeric wild type Aβ (1-42) at 5 µM concentration when compared to the untreated cells.
d) BV-2 cells were treated with freshly prepared mutant Aβ (1-40;G29,33I) and Aβ (1-42;G29,33I), which contained more monomers as the peptides were pretreated with hexafluoro isopropanol (HFIP).
   Treatment of BV-2 cells with freshly suspended monomeric mutant Aβ (1-40;G29,33I) and Aβ (1-42;G29,33I) peptides showed a maximum of 10% toxicity with the monomeric mutant Aβ (1-40;G29,33I) at 5 µM concentration and a maximum of 26% toxicity with monomeric mutant Aβ (1-42;G29,33I) at 5 µM concentration when compared to the untreated cells.
   These toxicities exerted by monomeric mutant peptides are slightly weaker than that of the monomeric wild type peptides (item c) above). This implies that the two C-terminal substitutions at positions 29 and 33 reduces the toxic effect of the peptides.
e) N2A cells were treated with freshly prepared wild type Aβ (1-40) and Aβ (1-42). Prior to treatment, the peptides were maintained under conditions which triggered oligomerization. Treatment of N2A cells with freshly suspended oligomeric wild type Aβ (1-40) and Aβ (1-42) peptides showed a maximum of 38% toxicity with oligomeric wild type Aβ (1-40) at 5 µM concentration and a maximum of 54% toxicity with oligomeric wild type Aβ (1-42) at 5 µM concentration when compared to the untreated cells.
f) N2A cells were treated with freshly prepared mutant Aβ (1-40;G29,33I) and Aβ (1-42;G29,33I). Prior to treatment, the peptides were maintained under conditions which triggered oligomerization.
   Treatment of N2A cells with freshly suspended oligomeric mutant Aβ (1-40;G29,33I) and Aβ (1-42;G29,33I) peptides showed a maximum of 25% toxicity with oligomeric mutant Aβ (1-40;G29,33I) at 5 µM concentration and a maximum of 16% toxicity with oligomeric mutant Aβ (1-42;G29,33I) at 5 µM concentration when compared to the untreated cells. The toxicities exerted by oligomeric mutant peptides are comparitively weaker than that of the oligomeric wild type peptides (item e) above). This implies that the two C-terminal substitutions at positions 29 and 33 minimize the toxic effect of the peptides.
g) BV-2 were treated with freshly prepared wild type Aβ (1-40) and Aβ (1-42). Prior to treatment, the peptides were maintained under conditions which triggered oligomerization. Treatment of BV-2 cells with freshly suspended oligomeric wild type Aβ (1-40) and Aβ (1-42) peptides showed a maximum of 26% toxicity with oligomeric wild type Aβ (1-40) at 5 µM concentration and a maximum of 53% toxicity with oligomeric wild type Aβ (1-42) at 5 µM concentration when compared to the untreated cells.
h) BV-2 cells were treated with freshly prepared mutant Aβ (1-40;G29,33I) and Aβ (1-42;G29,33I). Prior to treatment, the peptides were maintained under conditions which triggered oligomerization.
   Treatment of BV-2 cells with freshly suspended oligomeric mutant Aβ (1-40;G29,33I) and Aβ (1-42;G29,33I) peptides showed a maximum of 23% toxicity with oligomeric mutant Aβ (1-40;G29,33I) at 5 µM concentration and a maximum of 10% toxicity with oligomeric mutant Aβ (1-42;G29,33I) at 5 µM concentration when compared to the untreated cells. The toxicities exerted by oligomeric mutant peptides are comparitively weaker than that of the oligomeric wild type peptides (item g) above). This implies that the two C-terminal substitutions at positions 29 and 33 minimize the toxic effect of the peptides.

The toxicity assays show that the wild type and the mutant peptides are low-toxic in the freshly prepared-monomerized conditions both on the murine neuronal cell line N2A and on the microglial cell line BV-2.
Oligomerization of wild type Aβ (1-40) and Aβ (1-42) peptides exerted toxicity over both cell lines, N2A and BV2. Surprisingly, the double mutants Aβ (1-40;G29,33I) and Aβ (1-42;G29,33I) have a significantly reduced toxic effect on the cells in the same oligomerization conditions.

## Claims

1. A Polypeptide having an amino acid sequence comprising at least two substitutions of the glycine residues in one or more GXXXG motives in the amino acid sequence of the wild type Aβ peptide as depicted in SEQ ID NO:1.

2. The polypeptide according to claim 1, wherein the substitutions occur at the glycine residues at positions 29 and 33 of the wild type amino acid sequence of the Aβ peptide as depicted in SEQ ID NO:1.

3. The polypeptide according to claim 1 or 2, wherein the glycine residues can be replaced by hydrophobic amino acid residues, in particular by isoleucine.

4. The polypeptide according to any one of claims 1-3, having a sequence according to SEQ ID NOs: 2-7.

5. The polypeptide according to one of the preceding claims further comprising moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids.

6. A method for diagnosing a neurodementing disease, the method comprising the following steps:
a) incubating a polypeptide according to any one of claims 1 to 5 immobilized on a carrier with a sample derived from a subject, subsequently
b) separating said sample from the carrier, and
c) detecting polypeptides, preferably Aβ autoantibodies, bound to said immobilized polypeptide of step a).

7. A method for diagnosing a neurodementing disease, the method comprising the following steps:
a) incubating a polypeptide according to any one of claims 1 to 5 with a sample derived from a subject, and subsequently
b) incubating a carrier with the sample, the carrier having a binding affinity for said polypeptide according to any one of claims 1 to 5,
c) separating said sample from the carrier, and
d) detecting polypeptides, preferably Aβ autoantibodies, bound to said polypeptide according to any one of claims 1 to 6, said polypeptide being bound to the carrier.

8. The method according to any one of claims 6 or 7, wherein the detection of polypeptides bound to said polypeptide according to any one of claims 1 to 5 in step c) and d), respectively, is performed by means of ELISA, ELISPOT, Western-Blot, Dot Blot, Protein-Chip, surface plasmon resonance assays, isothermal caloriemetry, immunoprecipitation or co-immunoprecipitation, or affinity chromatography, in particular immunoaffinity chromatography.

9. A method for diagnosing a neurodementing disease, the method comprising the following steps:
a) incubating a polypeptide according to any one of claims 1 to 5 immobilized on a carrier with a cell containing sample derived from a subject,
b) separating said sample from the carrier, and
c) detecting cells bound to said polypeptide of step a).

10. A method for diagnosing a neurodementing disease, the method comprising the following steps:
a) incubating a polypeptide according to any one of claims 1 to 5 with a cell containing sample derived from a subject, and subsequently
b) incubating the sample with a carrier having a binding affinity for said polypeptide according to any one of claims 1 to 5,
c) separating said sample from the carrier, and
d) detecting cells bound to said polypeptide according to any one of claims 1 to 6, said polypeptide being bound to the carrier.
5

11. The method according to any one of claims 6, 7, 9, or 10, wherein at least one washing step is performed before the detecting step.

12. The method according to any one of claims 6, 7, 9, or 10, wherein said neurodementing disease is selected from Alzheimer's disease, Down's syndrome, Dementia with Lewy bodies, fronto-temporal dementia, cerebral amyloid angiopathy, Prion disease and/or amyloidoses.

13. A kit for diagnosing a neurodementing disease, wherein the kit comprises the polypeptide according to any one of claims 1 to 5.

14. The polypeptide according to the claims 1 to 5 for use as a medicament in human and veterinary medicine and as a diagnostic substance.

15. The polypeptide according to the claims 1 to 5 for use as a medicament for the prevention and for the diagnosis of the progression of Alzheimer's disease, Down's syndrome, Dementia with Lewy bodies, fronto-temporal dementia, cerebral amyloid angiopathy, and amyloidoses.


SEQUENCE LISTING

<110>  Balakrishnan, Karthikeyan
       Bacher, Michael
       Dodel, Richard

<120>  Diagnosis, Prophylaxis and Therapy of Alzheimer's Disease and
       other neurodementing disorders

<130>  MBA-001 EP

<140>  not yet known
<141>  2010-07-08

<160>  7

<170>  PatentIn version 3.3

<210>  1
<211>  42
<212>  PRT
<213>  Homo sapiens

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ala Ala
        35                  40

<210>  2
<211>  39
<212>  PRT
<213>  Artificial

<220>
<223>  synthetic sequence

<400>  2

Asp Ala Glu Phe Arg His Asp Ser Gly Tyr Glu Val His His Gln Lys
1               5                   10                  15

Leu Val Phe Phe Ala Glu Asp Val Gly Ser Asn Lys Ile Ala Ile Ile
            20                  25                  30

Ile Leu Met Val Gly Gly Val
        35

<210>  3
<211>  40
<212>  PRT
<213>  Artificial

<220>
<223>  synthetic sequence

<400>  3

Asp Ala Glu Phe Arg His Asp Ser Gly Tyr Glu Val His His Gln Lys
1               5                   10                  15

Leu Val Phe Phe Ala Glu Asp Val Gly Ser Asn Lys Ile Ala Ile Ile
            20                  25                  30

Ile Leu Met Val Gly Gly Val Val
        35                  40

<210>  4
<211>  41
<212>  PRT
<213>  Artificial

<220>
<223>  synthetic sequence

<400>  4

Asp Ala Glu Phe Arg His Asp Ser Gly Tyr Glu Val His His Gln Lys
1               5                   10                  15

Leu Val Phe Phe Ala Glu Asp Val Gly Ser Asn Lys Ile Ala Ile Ile
            20                  25                  30

Ile Leu Met Val Gly Gly Val Val Ala
        35                  40

<210>  5
<211>  42
<212>  PRT
<213>  Artificial

<220>
<223>  synthetic sequence

<400>  5

Asp Ala Glu Phe Arg His Asp Ser Gly Tyr Glu Val His His Gln Lys
1               5                   10                  15

Leu Val Phe Phe Ala Glu Asp Val Gly Ser Asn Lys Ile Ala Ile Ile
            20                  25                  30

Ile Leu Met Val Gly Gly Val Val Ala Ala
        35                  40

<210>  6
<211>  43
<212>  PRT
<213>  Artificial

<220>
<223>  synthetic sequence

<400>  6

Asp Ala Glu Phe Arg His Asp Ser Gly Tyr Glu Val His His Gln Lys

1               5                   10                  15

Leu Val Phe Phe Ala Glu Asp Val Gly Ser Asn Lys Ile Ala Ile Ile
            20                  25                  30

Ile Leu Met Val Gly Gly Val Val Ala Ala Thr
        35                  40

<210>  7
<211>  47
<212>  PRT
<213>  Artificial

<220>
<223>  synthetic sequence

<400>  7

Asp Ala Glu Phe Arg His Asp Ser Gly Tyr Glu Val His His Gln Lys
1               5                   10                  15

Leu Val Phe Phe Ala Glu Asp Val Gly Ser Asn Lys Ile Ala Ile Ile
            20                  25                  30

Ile Leu Met Val Gly Gly Val Val Ala Ala Thr Val Ile Val Ile
        35                  40                  45